# EUROPEAN PATENT APPLICATION

(11) **EP 2 937 075 A1**
(43) Date of publication of application: **28.10.2015**
(21) Application number: 13865534.5
(22) Date of filing: 20.12.2013
(51) Int. Cl.: A61K 8/60, A61K 8/14, A61Q 19/00, A61Q 19/08

(54) **SKIN BARRIER PROTECTIVE INGREDIENT AND COSMETIC AND/OR DERMATOLOGICAL FORMULATION CONTAINING THE SAME**

(30) Priority: 21.12.2012 BR 1232898
(71) Applicant: Botica Comercial Farmacêutica Ltda., 83050-010 São José dos Pinhais (BR)
(72) Inventor: CARVALHO, Camila Miranda de, 80730-080 Curitiba - PR (BR); FEFFERMAN, Israel Henrique Stokfisz, 80240-100 Curitiba - PR (BR); NICHELE, Tammy Proença Zagonel, 81720-072 Curitiba - PR (BR); SWINKA, Bruna Bastos, 80320-050 Curitiba - PR (BR); AFORNALI, Alessandro, 82300-310 Curitiba (BR); BROHEM, Carla Abdo, 80320-050 Curitiba - PR (BR); BAPTISTA, Marcela Contador, 80510-250 Curitiba - PR (BR); LORENCINI, Márcio, 80620-270 Curitiba - PR (BR); DIEMANT, Gustavo de Campos, 81200-100 Curitiba - PR (BR)
(74) Representative: McPherson, Michael
(86) International application number: PCT/BR2013/000590
(87) International publication number: WO 2014/094101

(57) **Abstract**

The present invention describes a protective ingredient of skin barrier formulation and a cosmetic and/or dermatological formulation containing the same which, in accordance with its general characteristics, has as basic principle the provision of a protective ingredient of skin barrier from a fraction derived from apple extract of *Malus sp* incorporated into liposomes (soy lecithin) in specified concentrations, in order to provide such lipossomed fraction of apple extract of *Malus sp* a different moisturizer activity that provides skin protection and minimizes the signs of aging, through reinforcement and improvement actions of the skin barrier function, induction of proliferation and differentiation of skin cells (keratinocytes), epidermal development, development of cell regulation and differentiation, favoring the blood circulation and the activation of cutaneous ceramides metabolism.

## Description

The present invention relates to ingredients for cosmetic and/or dermatologic products in general, and more specifically to a skin barrier protective ingredient and a cosmetic and/or dermatologic formulation containing the same which according to their general characteristics has as basic principle to provide a skin barrier protective ingredient from a fraction from the extract of apple *Malus sp* incorporated into liposomes (soy lecithin) in specific concentrations, in order to provide to such lipossomic fraction of apple *Malus sp* extract a differentiated moisturizer activity which provides protection to the skin and minimizes the signs of aging through reinforcing and improvement actions of skin barrier, induction of proliferation and differentiation of epidermal cells (keratinocytes), epidermal development, regulation of cell development and differentiation, favoring of blood circulation and activation of the skin ceramide metabolism.

Note that typically the number of divisions that a cell can suffer is limited, and this phenomenon is widely known as Hayflick limit. At each cell division the telomeres are shortened until they reach a critical length when cell proliferation can be stopped and the cell reaches senescence or aging.

The decrease in the regeneration potential of the tissue is one of the main causes of aging. Once the regeneration process of an organism is determined by the proliferative capacity, aging is directly dependent on the proliferation ability of skin cells. Additionally, cell renewal is a known mechanism for maintaining the homeostasis of the skin that involves keratinocyte proliferation and differentiation. With aging, the ability to regenerate the skin decreases. Thus, the stimulation of keratinocyte proliferation is a broad concern approach for the treatment of aging skin, promoting skin barrier enhancement.

In this context, it has become indispensable to structure an asset for skin application in different types of products, for example, soaps, shampoos, lipsticks and makeup in general, sunscreens lotions and creams, indicated primarily for the protection of skin barrier function, minimizing some of the major signs of aging, i.e., enabling to give various kinds of cosmetic and/or dermatologic products with proven application, constituting a novel option for human skin treatment.

At the same time, the demand for all kinds of plant ingredients has grown quite significantly in recent years, focusing on vegetables inputs that perform various functions related to human skin. Mechanisms of action related to the proliferation and differentiation, vitality and skin barrier function are of great interest in the cosmetic and dermatological areas, since they directly reflect the proper functioning and maintenance of the skin tissue of humans.

In this line, the apple extracts have been widely applied for a long time in cosmetic and dermatologic products from the skin care and makeup categories, for example, creams, lotions, soaps, serums, shampoos and sunscreens among many others.

In the specific case, the apple species *Malus domestica* is widely known in the art for its excellent longevity and ability to remain stable over time. From its tree, a Swiss apple tree known by the name *Uttwiler Spätlauber*, several portions of tissue were removed in order to obtain plant tissue cultures that allow broad studies of its composition and its properties.

In a broad review of the literature in order to establish the current state of art regarding protective ingredients of skin barrier object of the present patent, no relevant documents to the state of the art were disclosed correlating the specific object claimed in the present patent, i.e., a protective cosmetic and/or dermatological ingredient of skin barrier from a fraction derived from apple extract of *Malus sp* incorporated into liposomes (soy lecithin) in specified concentrations. However, secondary documents were disclosed that fit the references although they do not impede the present patent, in which:
- French patent document FR2916972 comprises combining apple extract, apple vinegar, almond glycoproteins, silicone and at least one essential oil. The composition and cosmetic products containing said combination are intended solely to inhibit the action of metalloproteinases, thereby preventing the degradation of elastin fibers in the dermal matrix, which departs from the object claimed in the present patent as it constitutes a distinctive combination of ingredients containing the apple extract.
- US patent document US20070036742 comprises the combination of extracts of one or more of the following plants: *Boswellia Serrata, Undaria Pinnatifida,* green tea, *Pureraria Mirifica,* luteolin, astilbin, vitamin E, amentoflavone, tetrahydropiperine, licochalcone, astaxanthin, red clover, *Brassica Juncea,* CoQI0 enzyme, sage, shikimic acid, oleo-resin of hop, apple, soy, ellagic acid, or any derivative thereof. The composition and cosmetic products containing said combination are intended solely to modulate the growth or regrowth of human hair.

In view of the documents of the state of the art mentioned above, it is clear that it is not provided a fraction from the apple extract *Malus domestica,* and the cosmetic and/or dermatological formulation containing said fraction is applied in the protection of the skin barrier function, working on specific cell and tissue mechanisms related to the skin, i.e., keratinocyte proliferation activities, improvement of skin barrier function and increased cell metabolism.

Thus, the patent in question is characterized by gathering components in a different design, which will meet the various requirements that the nature of the use demands, i.e., impart broad improvement of the skin barrier function providing protection to skin and minimizing some of the main signs of aging, besides acting in increasing the metabolism of lipids. Such design ensures that an ingredient protector of the skin barrier is efficient and reliable due to the aggregate technical qualities which provide advantages and improvements to the maintenance of the general balance of the human skin.

In this conception, the present protective ingredient of skin barrier and the cosmetic and/or dermatological formulation containing the same is based on a fraction from the extract of apple *Malus sp,* of Swiss origin, capable of acting directly on specific cell and tissue mechanisms related to the aging of human skin, i.e., which ensures a broad anti-aging activity and a perfect protection of the skin barrier function.

More specifically, a fraction from the extract of *Malus sp* consisting of arabinose, mannose, galactose and inositol defined concentrations was obtained by specific filtration processes, and this fraction was incorporated into a liposome concentrate derived from soybean phospholipids (lecithin), so that it became lipossomed, that is, protected by layers of phospholipids that allow its controlled release.

Note that this fraction is closely related to actions of inducing the cell proliferation and improving the skin barrier function, besides acting in increasing the metabolism of lipids, i.e., it is contemplated the offer of a composition containing the fraction obtained from *Malus domestica* able to act in different cell and tissue mechanisms of the human skin, in addition to having high stability when applied in cosmetic and/or dermatological formulations.

The present invention patent of "Protective Ingredient of Skin Barrier and Cosmetic and/or Dermatologic Formulation Containing the Same" comprises a set of cosmetic and/or dermatological solutions properly embedded, constituting an protective ingredient of skin barrier, the basic formulation of which is consistent with final differentiated qualities that, by their own characteristics, is obtained from a fraction derived from the apple extract of *Malus sp* consisting of arabinose, mannose, galactose and inositol in concentrations defined through specific filtration processes, and incorporated into a concentrate of liposomes derived from soybean phospholipids (lecithin) so that it became lipossomed, being the formulation and consequently the amounts employed based directly on the applications to which they are intended in order to allow obtaining an excellent protective ingredient of skin barrier that confers broad and effective overall balance to the skin and mitigates the aging by various cosmetic and/or dermatological formulations.

The "Protective Ingredient of Skin Barrier and Cosmetic and/or Dermatologic Formulation Containing the Same" comprises cosmetic and/or dermatological formulations in which their basic formulations consist of a range of about one hundredth percent to ten percent by weight of the protective ingredient of skin barrier.

The "Protective Ingredient of Skin Barrier and Cosmetic and/or Dermatologic Formulation Containing the Same" comprises cosmetic and/or dermatological formulations wherein said protective ingredient of skin barrier will eventually be added, and it may include ingredients constituent of the base composition or other active or functional ingredients such as surfactants, humectants, thickeners, fragrances, preservatives, sequestrants, sunscreens, scents, dyes, emulsifiers, active ingredients, sensory modifiers among others.

Considering the features and benefits described above, tests were carried out by various methodologies for evidence of the benefits and effectiveness of the ingredient of said formulations that contain the protective ingredient of skin barrier object of the present patent. The protocols used and results related to these parameters, as well as the formulations, are showed as examples in the following.

The cytotoxicity of the apple extract fraction was investigated using the MTS technique (a tetrazole compound), and the evaluation of the mechanism of cell death was by flow cytometry, by quantifying the specific protein markers. These tests indicated the absence of cytotoxicity in human skin fibroblasts until the maximum concentration tested, besides the absence of significant mechanisms of apoptosis or necrosis in the concentrations evaluated. Thus, the absence of processes related to the cytotoxicity to said fraction of apple extract was evidenced.

Afterwards, the effectiveness of the apple extract fraction was investigated in the proliferation of keratinocytes using the technical evaluation of keratinocyte proliferation rate in cell culture, after forty-eight hours in the presence or absence of the apple extract fraction. The proliferative capacity in reconstituted skin was also evaluated to twenty-one days for visualization of the number of layers in the epidermis. The results of biological tests showed that the apple extract fraction induced a significant increase in keratinocyte proliferation of about forty percent. In the reconstituted skin, the condition subject to said fraction had increased from 15 ± 2 to 20 ± 2 epidermal layers. Regarding the proliferation of keratinocytes *in vitro,* there was a significant increase (p ≤ 0:05) of the number of cells after a contact of forty-eight hours with the fraction. These results demonstrated that the apple extract fraction is capable of inducing an increase in keratinocyte proliferation, enhancing skin barrier function. Such results reinforce the role of the apple extract fraction in maintaining the skin structure.

Additionally, skin fragments *ex vivo* treated with the apple extract fraction were used for twenty-four hours for DNA microarray analyzes. The microarray results indicated that six hundred thirty-eight genes were modulated by the apple fraction extract, which are involved in the following biological activities: epidermal differentiation, cell growth, keratinocyte differentiation, development and regulation of cell differentiation metabolism of sphingolipids.

As a non-limiting example of cosmetic and/or dermatological formulations containing the protective ingredient of skin barrier object of the present invention patent, the cosmetic and/or dermatological formulations are presented for protective bases of skin barrier for lipstick or makeup in general, shower gel, facial / body / protective lotion and makeup base through the detailed tables below, in which the components are combined and the quantities represented in percent by weight based on the total weight of the composition.

### Example 1 - Lipstick

| CATEGORY | INGREDIENT | QUANTITY |
|---|---|---|
| Emollients | Acetylated lanolin alcohol Low molecular weight fatty alcohols Silicones Vegetable oils | 20-45% |
| Consistency agents | High molecular weight fatty alcohols Vegetable waxes Synthetic waxes | 10-25% |
| Humectants | Glycols Polyethylene glycols Derived from sucrose | 0.5-2.5% |
| Antioxidants | Natural (vitamins A and E) Synthetic (BHT, BHA) | 0.01-1% |
| Dyes | Natural pigments (iron oxides) Synthetic pigments | 0.05-5% |
| Active | Malus Domestica Fruit Cell Culture Extract (Factor 111) | 0.01-10.0% |
| Sequestrants/ Chelating agents | EDTA Gallates | 0.1-0.8% |
| Emulsifiers | Sorbitan derivatives Ethoxylated alcohols Sucrose esters Esters of fatty alcohols | 1.5-3.5% |
| Active | Vitamins Moisturizers Sunscreens | 0.02-2.5% |
| Preservatives | Benzyl alcohol Parabens Phenoxyethanol | 0.01-2% |
| Organoleptic agents | Fragrance | 0.02-1.5% |

### Example 2-Shower Gel

| CATEGORY | INGREDIENT | QUANTITY |
|---|---|---|
| Anionic surfactants Foaming agents | Sulfated fatty alcohols Sulfated and ethoxylated fatty alcohols Succinated fatty alcohols | 20-50% |
| Non-ionic surfactants Foam stabilizer Consistency donors | Amidated coconut derivatives Ethoxylated coconut derivatives | 2-10% |
| Humectants | Glycols Polyethylene glycols Derived from sucrose | 0.5-2.5% |
| Antioxidants | Natural (vitamins A and E) Synthetic (BHT, BHA) | 0.01-1% |
| Dyes | Synthetic pigments | 0.05-5% |
| Sequestrants/ Chelating agents | EDTA Gallates | 0.1-0.8% |
| Amphoteric surfactants Foam stabilizer and formulation | Coconut amphoteric agents Corn amphoteric agents | 1.5-10% |
| Active | Vitamins Moisturizers Sunscreens | 0.02-2.5% |
| Preservatives | Benzyl alcohol Parabens Phenoxyethanol | 0.01-2% |
| Thickeners Pearly effect | Polyethylene glycols derivatives Medium chain fatty alcohols | 0.2-2% |
| Active | Malus Domestica Fruit Cell Culture Extract (Factor III) | 0.01-10.0% |
| Thickeners | Carbomers Cellulose derivatives Polyacrylamides | 0.2-2% |
| Organoleptic agents | Fragrance | 0.02-1.5% |

### Example 3 - Facial Cream / Body / Shield

| CATEGORY | INGREDIENT | QUANTITY |
|---|---|---|
| Vehicle | Demineralized water | q.s. |
| Sequestrants/ Chelating agents | Disodium EDTA and/or others | 0.01-0.2% |
| Thickeners | Carbomers Cellulose derivatives Polyacrylamides | 0.1-2% |
| Humectants Moisturizers Active | Glycols Polyethylene glycols Derived from sucrose Hyaluronates Alpha hydroxy acids | 0.2-10% |
| Emulsifiers | Sorbitan derivatives Ethoxylated fatty alcohols Sucrose esters Esters of fatty alcohols | 0.1-5% |
| Consistency agents | High molecular weight fatty alcohols Vegetable waxes Synthetic waxes Vegetable oils | 0.5-5% |
| pH adjusters | Triethanolamine Aminomethyl propanol Sodium hydroxide | Qs |
| Emollients | Acetylated lanolin alcohol Low molecular weight fatty alcohols Silicones Vegetable oils Short and medium chain triglycerides | 1-15% |
| Active | Vegetable extracts Biotechnological extracts Vitamins Vegetable oils Peptides Others | 0.01-10% |
| Active | Malus Domestica Fruit Cell Culture Extract (Factor III) | 0.01-10.0% |
| Organoleptic agents | Fragrance | 0.1-0.5% |
| Preservatives | Phenoxyethanol Imidazolidinyl urea Others | 0.2-1.2% |

### Example 4: Base makeup

| CATEGORY | INGREDIENT | QUANTITY |
|---|---|---|
| Aqueous phase thickeners | Carbomers Cellulose derivatives Polyacrylamides | 0.1-2% |
| Emollients | Acetylated lanolin alcohol Low molecular weight fatty alcohols Silicones Vegetable oils | 2-20% |
| Consistency agents | High molecular weight fatty alcohols Vegetable waxes Synthetic waxes | 0.1-3% |
| Humectants | Glycols Polyethylene glycols Derived from sucrose | 0.5-10% |
| Antioxidants | Natural (vitamins A and E) Synthetic (BHT, BHA) | 0.01-1% |
| Dyes | Natural pigments (iron oxides) Synthetic pigments | 0.01-10% |
| Sequestrants/ Chelating agents | EDTA Gallates | 0.1-0.8% |
| Active | Vegetable extracts Biotechnological extracts Vitamins Vegetable oils Peptides Others | 0.01-10% |
| Emulsifiers | Sorbitan derivatives Ethoxylated fatty alcohols Sucrose esters Esters of fatty alcohols | 1.5-3.5% |
| Active | Malus Domestica Fruit Cell Culture Extract (Factor III) | 0.01-10.0% |
| Active | Vitamins Moisturizers Sunscreens | 0.02-15% |
| Preservatives | Benzyl alcohol Parabens Phenoxyethanol | 0.01-2% |
| Organoleptic agents | Fragrance | 0.02-1.5% |

For all the foregoing, it is an protective ingredient of skin barrier that will be well received by the users of cosmetic and/or dermatological formulations in general, because the present protective ingredient of skin barrier and cosmetic and/or dermatological formulation containing the same have numerous advantages, such as: high stability, cost effective, directly applied to a wide range of cosmetic and/or dermatologic products, and be sure to have a protective ingredient of skin barrier and hence the cosmetic and/or dermatological formulations containing the same that meet the current rules and regulations and the basic necessary conditions to its implementation.

All these attributes enable to classify this protective ingredient of skin barrier and cosmetic and/or dermatological formulation containing the same as one versatile, efficient and safe ingredient to be applied in a wide range of cosmetic and/or dermatological formulations, independent of the general characteristics that they can present, not limited at any time to the representations described herein, and that it should be understood in its broader set of claims, i.e., not limited to the particular form disclosed, and that other forms are understood as included within the scope of the appended claims.

## Claims

1. "PROTECTIVE INGREDIENT OF SKIN BARRIER AND COSMETIC AND/OR DERMATOLOGIC FORMULATION CONTAINING THE SAME", **characterized in that** it comprises a set of cosmetic and/or dermatological solutions constituting a protective ingredient of skin barrier, the basic formulation obtained from a fraction derived from apple extract of *Malus sp* containing arabinose, mannose, galactose, inositol in specific concentrations and incorporated into a liposome concentrate derived from soy phospholipids (lecithin) so that it became lipossomed, being the formulation and consequently the amounts employed based directly on the intended applications and conferring effective attenuation of aging by various cosmetic and/or dermatological formulations.

2. "PROTECTIVE INGREDIENT OF SKIN BARRIER AND COSMETIC AND/OR DERMATOLOGIC FORMULATION CONTAINING THE SAME", according to claim 1 and **characterized in that** it comprises cosmetic and/or dermatological formulations in which the basic formulations consist of a range of about one hundredth percent to ten percent by weight of the protective ingredient of skin barrier.

3. "PROTECTIVE INGREDIENT OF SKIN BARRIER AND COSMETIC AND/OR DERMATOLOGIC FORMULATION CONTAINING THE SAME", according to claims 1 and 2, **characterized in that** it comprises cosmetic and/or dermatological formulations wherein said protective ingredient of skin barrier that will be eventually added may include ingredients constituent of the base composition or other active or functional ingredients such as surfactants, humectants, thickeners, fragrances, preservatives, sequestrants, sunscreens, scents, dyes, emulsifiers, active ingredients, sensory modifiers among others.
